# EUROPEAN PATENT SPECIFICATION

(11) **EP 2 918 675 B1**
(45) Date of publication and mention of the grant of the patent: **23.11.2016**
(21) Application number: 15386007.7
(22) Date of filing: 12.03.2015
(51) Int. Cl.: C12N 5/09, C12M 1/12

(54) **Gaseous plasma nanotextured substrates for selective enrichment of cancer cells**
NANOTEXTURIERTE SUBSTRATE VON GASFÖRMIGEM PLASMA ZUR SELEKTIVEN ANREICHERUNG VON KREBSZELLEN
SUBSTRATS NANOTEXTURÉS À PLASMA GAZEUX D'ENRICHISSEMENT SÉLECTIF DE CELLULES CANCÉREUSES

(30) Priority: 12.03.2014 GR 20140100142
(43) Date of publication of application: 16.09.2015
(73) Proprietor: National Center for Scientific Research "Demokritos" Neapoleos &, 15310 Athens (GR); Gogolides, Evangelos, 15310 Aghia Paraskevi Attikis (GR); Tserepi, Angeliki, 15310 Aghia Paraskevi Attikis (GR); Kakabakos, Sotirios E., 15310 Aghia Paraskevi Attikis (GR); Petrou, Panagiota, 15310 Aghia Paraskevi Attikis (GR)
(72) Inventor: Gogolides, Evangelos, 15310 Aghia Paraskevi Attikis (GR); Tserepi, Angeliki, 15310 Aghia Paraskevi Attikis (GR); Kakabakos, Sotirios, 15310 Aghia Paraskevi Attikis (GR); Petrou, Panagiota, 15310 Aghia Paraskevi Attikis (GR); Kanioura, Anastasia, 15310 Aghia Paraskevi Attikis (GR); Bourkoula, Athanasia, 15310 Aghia Paraskevi Attikis (GR)

(56) References cited:
- WO-A1-03/020872
- WO-A1-2007/031799
- WO-A1-2009/150479
- LEE J H ET AL: "Interaction of Different Types of Cells on Polymer Surfaces with Wettability Gradient", Journal of Colloid And Interface Science, January 1998 (1998-01), pages 323-330, XP055164516, DOI: 10.1006/jcis.1998.5688 Retrieved from the Internet: URL:http://www.sciencedirect.com/science/a rticle/pii/S0021979798956880
- TSOUGENI K ET AL: "Plasma Nanotextured PMMA Surfaces for Protein Arrays: Increased Protein Binding and Enhanced Detection Sensitivity", LANGMUIR, vol. 26, no. 17, 7 September 2010 (2010-09-07), pages 13883-13891, XP055164512, ISSN: 0743-7463, DOI: 10.1021/la101957w
- WANG S ET AL: "Three-Dimensional Nanostructured Substrates toward Efficient Capture of Circulating Tumor Cells", ANGEWANDTE CHEMIE INTERNATIONAL EDITION, vol. 48, no. 47, 9 November 2009 (2009-11-09), pages 8970-8973, XP055034516, ISSN: 1433-7851, DOI: 10.1002/anie.200901668

## Description

### Field of the Invention

The present invention relates to the field of isolation and enrichment of cancer cells when in mixture with normal cells.

### State of the art and background of the invention

In order to comprehend the interaction between cells and their underlying substrates, life science researchers are beginning to utilize micro- and nano-structured surfaces to study cellular behaviour. The most notable applications of such micro - nano-surface/cell interactions are in the fields of biosensors, biochips for cell microarrays and diagnostics, tissue engineering and medical implants for prosthesis (A. Khademhosseini, et al., Proceedings of the National Academy of Sciences of the United States, of America, 2006, 103, 2480-2487). In several of these fields a fundamental understanding of cell-substrate interactions is the key to the future development of biotechnology (A. V. Singh, et al., Journal of Biomedical Materials Research Part A, 2013, 101, 3019-3032).

Cell adhesion onto a substrate is highly affected by distinct surface properties such as topography, roughness, electrical charge, stiffness, functional groups, and wettability (A. V. Singh, et al., Journal of Biomedical Materials Research Part A, 2013, 101, 3019-3032).

Surface wettability has been reported as one of the critical factors that affect cell behavior. Ikada et al. modified several polymeric substrates with gaseous plasma and studied the effect of plasma treatment duration on cell adhesion (Y. T. a. Y. Ikada, Polymer, 1993, 34, 2208-2212). Although they observed a different dependence of cell adhesion on the duration of plasma treatment for each polymer in all cases, the optimal water contact angle for cell adhesion was approximately 70°. Furthermore, it was found, that cell attached predominantly on the plasma-treated surfaces than on the non-treated ones when compared at the same contact angle, i.e. the same water wettability.

Lee et al. created a gradient of wettability on polyethylene surfaces by corona discharge treatment and examined cell adhesion to the treated surfaces (J. H. Lee, et al., Journal of Colloid and Interface Science, 1998, 205; 323-330). They found that the maximum cell adhesion was achieved at the surface with a water contact angle of 55°, regardless of the cell type used.

Ruady et al. studied the adhesion of human skin fibroblasts on glass surfaces modified with dichlorodimethylsilane (DDS) to create a wettability gradient and reported that cells adhered preferably on the surface with water contact angles values around 50° (T. G. Ruardy, et al., Journal of Biomedical Materials Research, 1995,29,1415-1423).

Arima et al. created surfaces of different wettability through modification of glass surfaces with mixed self-assembled monolayers of alkanethiols with different terminal groups (Y. Arima, et al., Biomaterials, 2007, 28, 3074-3082). Using these surfaces, they evaluated the effects of surfaces wettability on the adhesion of human umbilical vein endothelial cells and HeLa cells. They found that both cell types adhered well on surfaces with water contact angles values ranging from 40 to 60°, and the number of adhered cells decreased as contact angle was increased from this point up.

Cell adhesion on superhydrophobic surfaces has been also studied. Senesi et al. reported increased cell adhesion upon increasing the contact angle from 115 to 160° on PET surfaces modified by fluorocarbon deposition through treatment by plasma enhanced-chemical vapour deposition of C₂F₄ (G. S. Senesi, et al., Surface Science, 2007, 601, 1019-1025).

However, other studies employing patterned surfaces with superhydrophobic and cell-culture treated regions suggested the opposite result, i.e., cells did not adhered and proliferated onto the superhydrophobic surfaces (K. Ino, et al., Journal of Bioscience and Bioengineering, 2007, 104,420-423).

Topography is another critical factor defining cell adhesion and proliferation behaviour. Cell adhesion behavior has been examined on nano-structured surfaces with various morphologies such as nanopillars (V. Hasirci, et al., 2012, J. W. Lee, et al., Analytical sciences, 2011, 27, 369-374), nanoposts (C. H. Choi, et al., Biomaterials, 2007, 28, 1672-1679); nanogrooves (H. W. Baac, et al., Materials Science & Engineering C-Biomimetic and Supramolecular Systems, 2004, 24, 209-212, D. W. Hamilton, et al., PLoS ONE, 2010, 5), ridges (A. I. Teixeira, et al., Journal of Biomedical Materials Research Part A, 2004, 71A, 369-376, E. K. F. Yim, et al., Biomaterials, 2010, 31, 1299-1306), pores and wells (M. S. Hahn, et al., Biomaterials, 2006, 27, 1104-1109).

In particular V. Hasirci et al in WO2013/162482 A1 teach how to create a matrix of squares; each square comprising micro pillars of different dimensions and spacing using e-beam lithography, to examine cell adhesion with respect to topography (V. Hasirci, et al., 2012). They examined two different cell types and found different behaviour with regard to pillar dimensions and spacing. In addition, the proposed structures were limited to microscale dimensions and they have not evaluated for specific cell type enrichment.

Lee et al. employed laser interference lithography and a nanoimprinting technique to create nanopatterns of different density (J. W. Lee, et al., Analytical sciences, 2011, 27, 369-374). They cultured human osteoblasts on the nanopatterned surfaces and studied filopodia protrusion. They found that cells preferred the low density pattern over the high density one. Surfaces with continuous or discontinuous nanogrooves fabricated using also laser interference lithography were employed to study the adhesion and growth of periodontal ligament fibroblasts on topographies (D. W. Hamilton, et al., PLoS ONE, 2010, 5). It was found, that the examined cells adhered to and spread on all tested surfaces, but less favourably on the surfaces with the discontinuous nanogrooves. They also found that filopodia elongation and migration was guided by the nanopatterns and the cells responded mainly to nanotopographies with depths of 85-100 mm. Choi et al. created two distinct nanopatterns, i.e. posts and grates, of various dimensions, periodicity and tip shape on which human foreskin fibroblasts were cultured (C. H. Choi, et al., Biomaterials, 2007, 28, 1672-1679). The cells exhibited smaller size and lower proliferation on needle like nanoposts, and enhanced elongation and alignment on blade-like nanogrates, with both results amplified by increase in the nanostructure height.

Baac et al., made use of laser holography (on a photo-responsive azobenzene copolymer layer) to produce gratings of a regular sinusoidal shape on the surface (H. W. Baac, et al., Materials Science & Engineering C-Biomimetic and Supramolecular Systems, 2004, .24, 209-212). Human astrocytes cultured on these surfaces adhered and elongated preferentially onto the patterned surface.

Yim et al. studied the growth of stem cells on nanotopography created by soft lithography and embossing on tissue culture polystyrene and polydimethylsiloxane (E. K. F. Yim, et al., Biomaterials, 2010, 31, 1299-1306). They suggested that both nanotopography and substrate stiffness are determining the mechanical properties of the adhered cells, while nanotopography determines mainly the organization of the cells cytoskeleton.

Hahn et al., used photoactive poly(ethylene glycol)-diacrylate hydrogels as model substrates and created 3D structures by photolithography. The surfaces were evaluated with respect to adhesion of human dermal fibroblasts (M. S. Hahn, et al., Biomaterials, 2006, 27, 1104-1109).

In some cases, wettability and nanotopography effects were combined on the same surface.

Khorasani et al. studied the attachment of baby hamster kidney fibroblasts on CO₂-pulsed laser modified polydimethylsiloxane with different hydrophobicities and random nanotopography (M. T. Khorasani, et al., Colloids and Surfaces B-Biointerfaces, 2004, 35, 67-71). It was found that cells adhesion and spreading was less on treated PDMS with rough porous surface than on the untreated, smooth and non-porous control surface.

Ko et al. explored the behaviour of mouse liver cancer cells on oxygen plasma, nanostructured PET surfaces in extreme wetting conditions (T. J. Ko, et al., Soft Matter, 2013, 9, 8705-8711). The mouse liver cancer cells proliferated significantly on a mild hydrophilic surface with a low aspect ratio nanostructure due to the improvement of mechanical anchoring. On the other hand, the superhydrophilic surface with a high aspect ratio nanostructure suppressed the growth of the cancer cells.

None of the above mentioned works discloses separation or enrichment of cancer cells from a mixture with normal cells. Selective capture and growth of cancer cells from mixture with normal cells has been achieved in the prior art either by employing appropriately structured microfluidics or surfaces modified with antibodies that recognize specific antigens on the cancer cell membrane.

In particular, microfluidics based on oscillatory flow have been applied to separate cells based on their size and deformability (S. M. McFaul, et al., Lab on a Chip, 2012, 12, 2369-2376, S. J. Tan, et al., Biomedical Microdevices, 2009, 11, 883-892). Another microfluidic device with deterministic lateral displacement arrays was also developed by Liu et al. in order to achieve cancer cell separation and enrichment from whole blood, by exploiting the size-dependent hydrodynanic forces (Z. B. Liu, et al., Biomicrofluidics, 2013, 7).

On the other hand, devices aiming to enrichment of cancer cells populations from mixtures with normal cells based on the specific interaction of cell membrane antigens with antibody or aptamer-functionalized surfaces have been also reported (Y. F. Huang, et al., Analytical Chemistry, 2008, 80, 567-572, S. Nagrath, et al., Nature, 2007, 450, 1235-U1210, S. L. Stott, et al., Proceedings of the National Academy of Sciences of the United States of America, 2010, 107, 18392-18397). Recent research used the higher fractality of structures determined on cancer cells compared to normal cells in order to achieve selective adhesion of cancer cells (P. C. Zhang, et al., Advanced Materials, 2013, 25, 3566-3570). Surfaces with Gold (Au) fractal nanostructures were prepared with electrochemical deposition and the nanostructures were modified with anti-EpCAM antibodies to specifically bind to epithelial cancer cells expressing EpCAM antigen on their membrane. However, when the nanotextured surfaces were not modified with the anti-EpCAM antibody, the efficiency of the fractal surfaces to capture cancer cells was improved only by a factor of approximately 3 compared to the respective flat surface(L. Chen, et al., Advanced Materials, 2011, 23, 4376-+, S. T. Wang, et al., Angewandte Chemie-International Edition, 2011, 50, 3084-3088, S. T. Wang, et al., Angewandte Chemie-International Edition, 2009, 48, 8970-8973). The use of surfaces modified with specific capture biomolecules, such as antibodies or aptamers results in efficient separation of cancer cells from biological fluids or from mixtures with other cells. However, the use of antibodies or specific capture molecules is complex and such molecules are expensive and prone to degradation. Thus, solutions towards cancer cell capture and enrichment based either exclusively on surface topography or on the combination of surface topography and its chemical/biological functionalization are highly desirable.

In this direction efforts have been made to develop microfluidic systems in combination with highly ordered micropillars on surfaces to achieve enrichment of cancer cells when in mixture with normal cells. Kwon et al. used capillary molding through a transparent, mold and UV (ultraviolet) light exposure to nanostructure a polyurethaneacrylate photocrosslinkable polymer film with 0.4μm line and space patterns, or 0.4μm posts. They then embedded this film as a fourth wall to seal a polydimethyl siloxane open microfluidic channel and achieved separation of human breast epithelial cells and cancer cells based on the different adhesion of these two types of cells for 2-h culture period and detachment by the channel surface under flow conditions (K. W. Kwon, et al., Lab on a Chip, 2007, 7, 1461-1468). Using this device an enrichment of cancer cells over normal cells from 0.36 to 0.83, from an initial 1:1 mixture, i.e. only by a factor of 2.3. It seems therefore that micropillars or submicron pillars (a few hundred nm in size) do not possess the appropriate size and morphology to achieve efficient cancer cell enrichment.

Our team has previously proposed plasma nanotexturing as a technology to fabricate high aspect ratio random nanostructures on polymers (A. Tserepi, et al., 2006, K. Tsougeni, et al., Langmuir, 2009, 25, 11748-11759) . (Greek patent. Application number: 20050100473, PCT publication number: WO2007031799). After the plasma treatment, polymers have a long-lasting hydrophilic chemical functionality comprising OH, COOH, CO and related groups on the nanostructures.

This technology was found very important for biomolecule adsorption and creation of intense and sensitive protein and DNA microarrays (K. Tsougeni, et al., Analytical and Bioanalytical Chemistry, 2012, 403, 2757-2764, K. Tsougeni, et al., Langmuir, 2010, 26, 13883-13891). In addition, the team has discovered how to tune the dimensions and randomness of the nanotexturing creating periodic surface nanotextures by modifying the plasma processing conditions (E. Gogolides, et al., 2009, N. Vourdas, et al., Nanotechnology, 2010, 21, 085302). (Greek patent Application number: 20080100404, PCT publication number: WO2009150479). Such surfaces were also used by our group to culture cells (3T3 line of immortalized mouse fibroblasts) and it was found that these cells adhered, but did not proliferate on nanotextured surfaces (D. Kontziampasis, et al., Proc. SPIE 8765, Bio-MEMS and Medical Microdevices, 2013, 87650B).

The identification of cancer cell in biological samples plays a very important role in the field of diagnostics. It is vital that the existence of such cancer cells is monitored when their numbers are very small, so that their harmful effects can be remedied. There is a need for diagnostic devices capable of performing such cancer cell enrichment and separation if possible without the use of specific antibodies targeting such cells.

### Summary of the invention

The present invention relates to the field of isolation and enrichment of cancer cells from mixtures with normal cells using nanotextured polymeric or polymer coated surfaces produced by treatment with gaseous plasma. The surfaces of the present invention exhibit high surface-area comprising hierarchical, random, or quasi-ordered, high-aspect ratio micro and nanostructured rough morphology. The hierarchically nanotextured surfaces provide enhanced adhesion and proliferation of cancer cells compared to the corresponding flat substrates whereas the proliferation of normal cells on the nanotextured surfaces is reduced.

### Brief description of the drawings

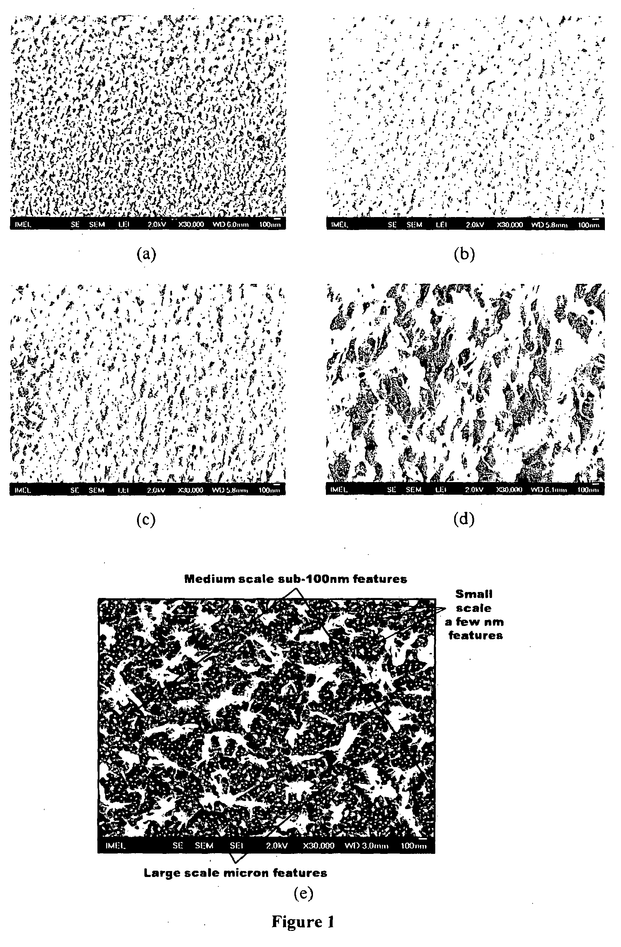

Figure 1a,b,c,d,e depicts Scanning Electron Microscopy (SEM) images of nanotextured surfaces of the present invention for varying bias voltage and plasma treatment time when the sample temperature was 15°C: The following are shown: (a) -50 V, (b) - 75 V, (c) -100 V for 1 min, (d) -100 V for 3 min, (e) top down view of the -100V for 3min etching, showing hierarchical nanotexture morphology and the spacing at the largest scale. Here three scale lengths are marked to aid the definition of hierarchical nanotexture morphology.

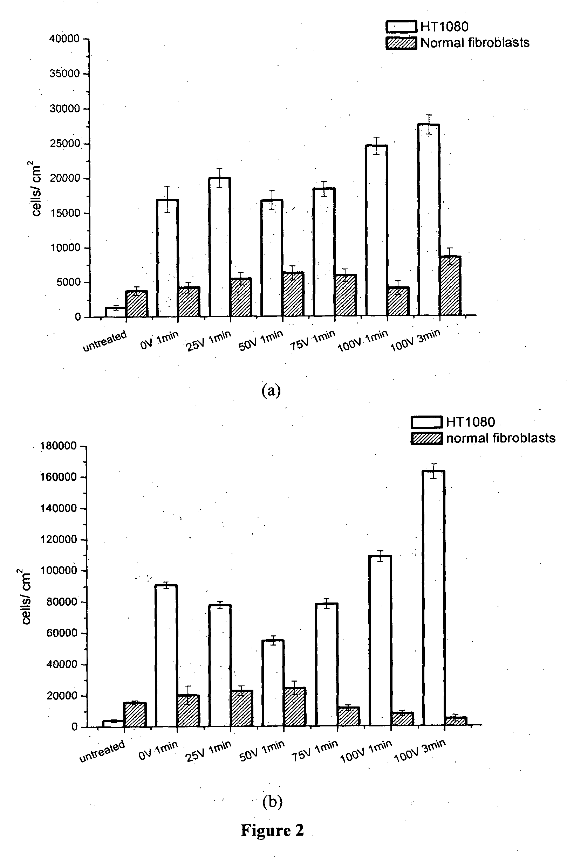

Figure 2a,b presents in columnar (bar format) the cancer and normal cell populations per surface area for untreated and O₂ plasma treated surfaces for a) 1 day and b) 3 days culture, respectively and for varying bias voltage and plasma treatment time (O₂ plasma, 0V, -25V, -50V -75V, -100V for 1 min and -100V for 3 min).

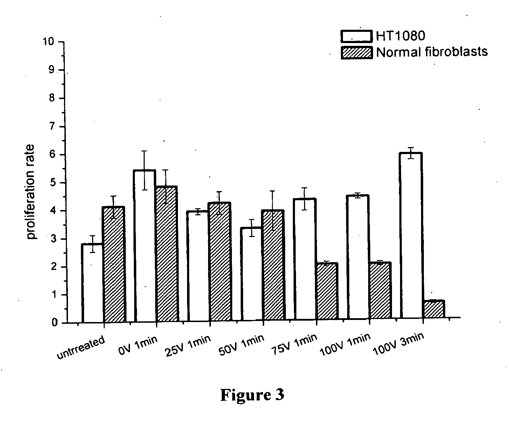

Figure 3 shows the proliferation rate of normal and cancer cells for untreated and O₂ plasma treated nanotextured surfaces. (Mean value ±SD, n=6, three different experiments in duplicate).

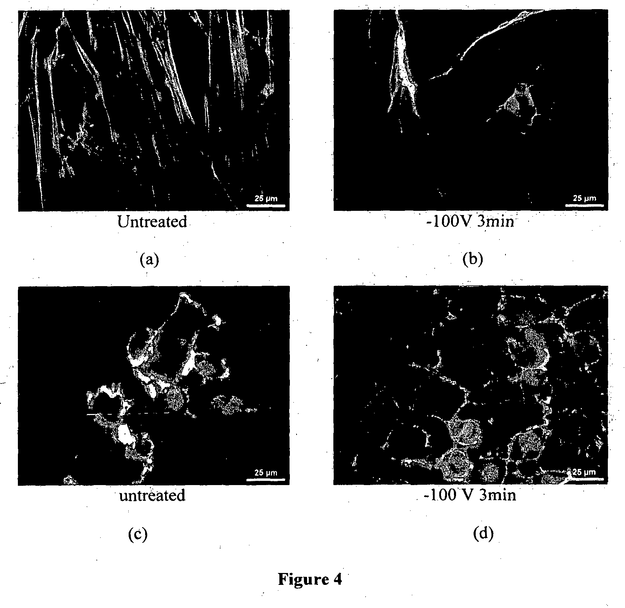

Figure 4a,b,c,d shows fluorescence microscope images (in black and white only) of (a,b) normal fibroblasts and (c,d) cancer cells on untreated (a,c) and plasma treated surfaces (b,d) after 3 days of culture. Cells were stained with Phalloidim-Atto 488 and DAPI for cytoskeleton and nucleus visualization, respectively.

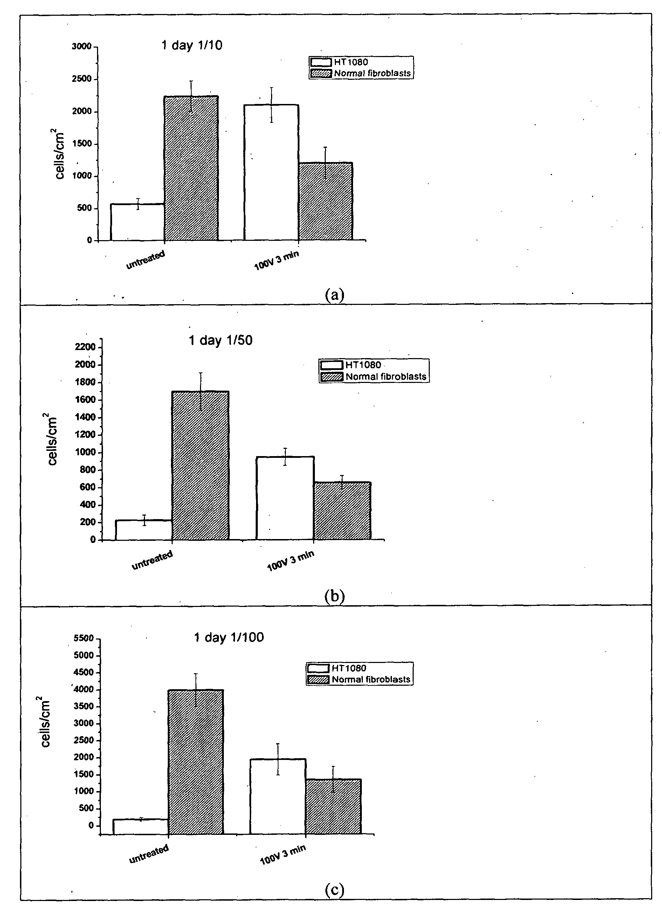

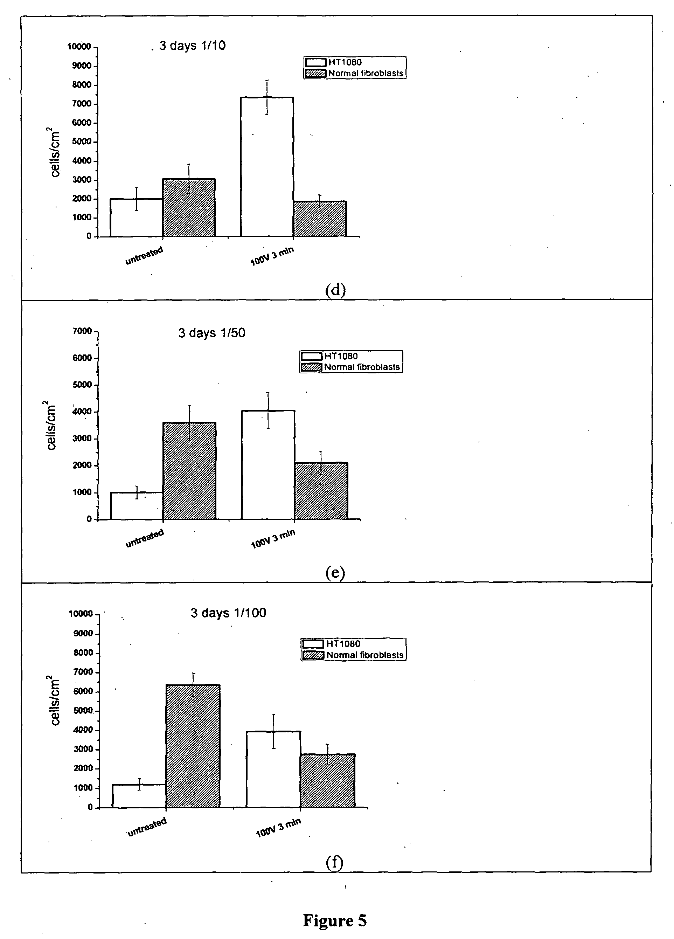

Figure 5a,b,c,d,e,f. shows the average number (± SD, n=6, three different days in duplicate) of physiological and cancer cells per surface area for untreated and oxygen plasma treated PMMA surfaces, for initial ratios of cancer to normal cells a) 0.1, b) 0.02 and (c) 0.01, for 1 day co-culture, while (d), (e), (f), show the corresponding results for 3 days of culture, demonstrating enrichment as higher than 140:1.

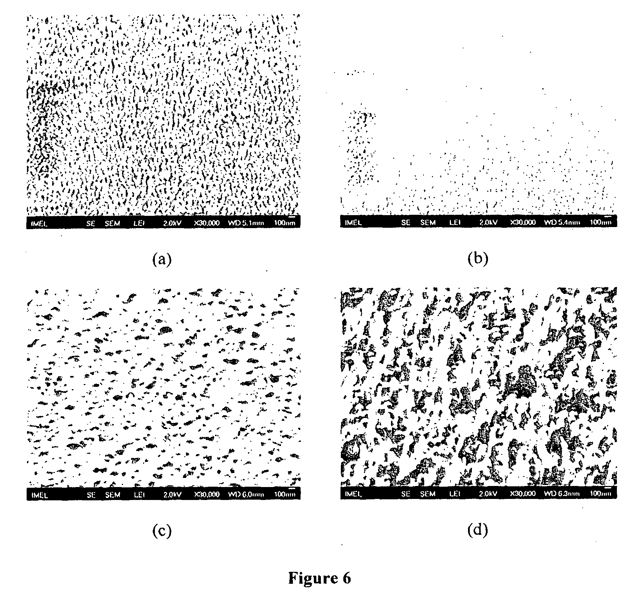

Figure 6a,b,c,d shows SEM images of PMMA surfaces treated with varying bias and plasma treatment time when the sample temperature is 65°C: (a) -50 V, (b) - 75 V, (c) -100 V for 1 min, and (d) -100 Volt for 3min.

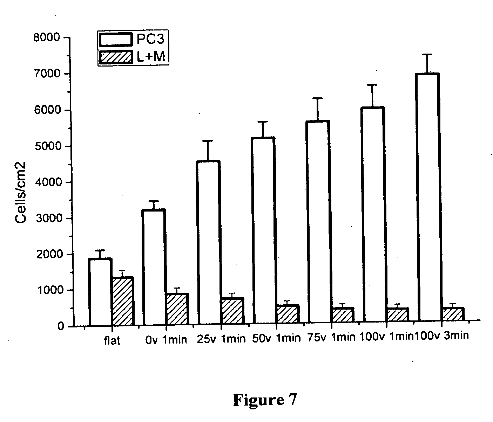

Figure 7 shows the average number of adhered PC3 cancer cells and monocytes per surface area for untreated and oxygen plasma treated nanotextured surfaces for one day of culture.

### Detailed description of the invention

The present invention provides selective adhesion, proliferation and enrichment of cancer cells from mixtures with normal cells using hierarchical random or quasi-ordered nanotextured polymeric or polymer coated surfaces. Surfaces of such morphology may be produced by various methods, such as gaseous plasma treatment (etching or deposition), electrochemical etching or deposition, laser ablation, nanoparticle deposition, and many others. In the present invention such surfaces are preferably produced by treatment with gaseous plasma.

Here the term hierarchical nanotexture is used to describe a rough surface with a composite mutliscale morphology of at least two length scales, comprising randomly or quasi-orderly positioned structures of micrometer or several hundred-nanometer size, which in turn comprise of smaller sub-hundred nanometer single-scale or composite multiscale structures, while such smaller single-scale or composite multiscale structures may exist also in the space between the larger structures. The surfaces are characterized by the height, width and spacing of the larger structures, while their aspect ratio (height over width) is larger than 3. The larger structures may be either random or quasi-ordered when they possess order over a length scale of several micrometers, but not over larger scale lengths. A typical plasma nanotextured surface is shown in Fig. 1e, where three scale lengths are shown, as well as the spacing among the large scale features.

According to the present invention the term "organic polymeric surface" means surfaces made of an organic polymer, as well as surfaces made of other materials, such as glass, metal, different polymer or other substrate, which are coated with an organic polymer film.

According to the present invention, the term "organic polymer" means a polymer containing carbon, oxygen and hydrogen. According to an embodiment of the invention the polymer further contains silicon atoms.

Preferably, the organic polymer of the present invention belongs to the group of acrylic copolymers, or olefin, or phenolic polymers, silicones or organosilicon polymers-organic copolymers. More preferably, the organic polymer is selected from the group consisting of poly ether ether ketone (PEEK), poly ethylene terepthalate (PET), cyclo olefin polymer (COP), cyclo olefin copolymer (COC), poly styrene (PS), poly dimethyl siloxane (PDMS), and PDMS-acrylate copolymers (see also our previous work (K. Tsougeni, et al., Analytical and Bioanalytical Chemistry, 2012 403, 2757-2764)). Even more preferably, the organic polymer is poly(methyl methacrylate) (PMMA).

The organic polymer is preferably used in the form of plates, or foils, or films spin-coated on flat surfaces such as Silicon wafers, metal, or glass slides.

The surfaces of the present invention are etched and simultaneously nanotextured in Oxygen containing plasmas. In the case of silicon containing polymers the plasma may in addition contain Fluorine. Under conditions of varying ion energy, processing time, and temperature, various hierarchical nanotexture geometry height, spacing and order may be produced. The etching and nanotexturing of the organic polymer surfaces may be carried out using a high density helicon or inductively coupled plasma reactor, or a reactive ion etcher, or other plasma etchers.

The etching has to be carried out in the presence of etching inhibitors. As disclosed in our previous inventions (Greek patent Application number: 20050100473, PCT publication number: WO2007031799) and (Greek patent Application number: 20080100404, PCT publication number: WO2009150479) the formation of nanotexture is caused by polymer etching with simultaneous deposition of unetchable inhibitors from the plasma reactor walls, or electrodes, or polymer etching which contains pre-existing such inhibitors in the polymer or on the polymer surface. In inductively coupled plasma reactors or helicon wave reactors if the
electrostatic fields are not shielded in the antenna, co-sputtering of dielectric material during etching creates etching inhibitors resulting in nanotexture formation (E. Gogolides, et al., Journal of Physics D-Applied Physics, 2011, 44). In Reactive ion etcher machines sputtering of electrode material creates etching inhibitors. Changing plasma parameters for a given plasma reactor- changes the nanotexture geometrical characteristics.

For the present invention, etching must be conducted under anisotropic etching conditions with typical operating pressures from 0.4-7 Pa and preferably 0.5-2Pa. According to the present invention the average hierarchical nanotexture height should be larger than 140 nm, preferably larger than 380 nm and more preferably larger than 800 nm, while the spacing should be larger than 100 nm and more preferably larger than 600 nm. Hierarchy should extend at least from sub-hundred to a few hundred nanometers, and preferably from a few nanometers to several hundreds of nanometers in size. For the present invention the height of the nanotexture of the polymeric surfaces may be controlled by varying the ion energy (i.e. the negative bias voltage applied to the sample and generated by a power generator), and for a specific energy by varying the duration of etching.

According to the present invention, using an unshielded inductively coupled plasma etcher, the desired hierarchical nanotexture is obtained for at least 1 min of etching, when the absolute value of the negative bias voltage is higher than 50V preferably higher than 75V, and more preferably higher than 100V. The duration of etching is preferably larger than 1min, and more preferably larger than 3min. In all cases the spacing between nanostructures is proportional to their height, and their morphology is hierarchical, the hierarchy extending to more scales with increasing processing time and bias voltage.

The order or randomness of the larger-scale nanostructures for the present invention may be controlled by the sample temperature during etching and the bias voltage. In particular higher temperatures (up to 20°C below the glass transition temperature of the polymer) and low bias voltages (less than 75V) produced quasi-ordered structures. On the contrary, lower temperatures (-100° to +40°C below the glass transition temperature), and higher bias voltages (higher than 75 V) produced random nanostructures. Preferably, the random nanostructures comprise taller than 380 nm, more complex and more hierarchical morphology.

It is important that after plasma treatment and before cell culturing surfaces should exhibit hydrophilicity with contact angles ranging from 0-70°, and preferably between 0-50°. The use of oxygen containing plasmas, and the nanotexture created ensures that such a stable in time contact angle is obtained (K. Tsougeni, et al., Langmuir, 2009, 25, 11748-11759).

It has now been found that surfaces treated as described above facilitate the adhesion of cancer cells in comparison to normal (non-cancerous) cells.

Furthermore, it has been found that surfaces treated as described above facilitate the proliferation of cancer cells in comparison to normal cells.

According to the present invention, the surfaces are not modified with antibodies that recognize specific antigens on the cancer cell membrane. This provides the advantages of low cost and stability of these surfaces in time. Nevertheless, such surfaces could also be used with an antibody taking advantage of their high surface area due to the hierarchical nanotexture.

Therefore, the present invention provides the use of an organic nanotextured polymeric surface without any antibody functionalization for the enrichment of cancer cells from mixtures containing cancerous and normal cells.

According to a preferred embodiment, the mixture containing cancerous and normal cells is a biological sample, such as a tissue sample taken from an animal or a human. More preferably, the mixture is a tissue sample containing normal and cancerous epithelial cells. For example, the mixture comprises cancerous epithelial skin cells, such as the Fibrosarcoma HT1080 cell line and normal fibroblasts. According to another preferred embodiment, the biological sample is a blood sample taken from an animal or a human. For example, the mixture is a blood simple containing prostate cancer cells, such as the PC3 line cells and isolated human blood mononuclear cells.

Preferably, the present invention achieves an enrichment of cancer cells by at least a factor of 10, and more preferably larger than a factor of 140.

The present invention also provides a diagnostic device for the enrichment of cancer cells in a mixture of cancer cells with normal cell. This diagnostic device may be used in a laboratory, or a clinic for enrichment and subsequent observation of cancer cells. Typical diagnostic devices of the present invention include but are not limited to microwells, microfluidics, microarrays, petri dishes etc.

### Examples

### Example 1: Effect of topography of O₂ plasma nanotextured PMMA surfaces on fibrosarcoma HT1080 cancer cells and normal cell adhesion, proliferation, and morphology

PMMA films were prepared by spin-coating on Si wafers a 25% w/w solution of Mw-120,000 PMMA (Sigma-Aldrich Co.) in 1-methoxy-2-propanol-acetate at 1000 rpm 30s, and baked for 1.5 h at 150 °C. These prepared PMMA films were nanotextured in O₂ plasmas in a Helicon Plasma reactor with unshielded electrostatic fiels (MET system, Adixen). The O₂ discharge process parameters were: 1900 W source power, 0.75 Pa pressure, 100 sccm oxygen flow, 0 to -100 V bias voltage, and sample temperature during etching 15 °C: In order to evaluate the adhesion and proliferation of cells on the nanotextured PMMA film surfaces, cancer cells from the fibrosarcoma cell line HT1080 and normal skin fibroblasts were used. The cells were cultured in Dulbecco's modified Eagle medium (DMEM) supplemented with 10% (v/v) fetal bovine serum, 2 mM L-glutamine, and 1% (v/v) penicillin/streptomycin in tissue culture dishes at a 37 °C incubator with humidified atmosphere of 5% CO₂ until the cell culture attained 70-80 % confluence. Then, the cells were treated with a trypsin-EDTA solution (0.05%/0.02% (w/v) in PBS) to detach the cells from the surface. The detached cells were appropriately diluted with DMEM culture medium and used to seed the PMMA surfaces.

The PMMA surfaces were sterilized by exposure to ultraviolet light for 20 min and then seeded with 5x10⁴ cells/ mL. Cells were cultured as described above for up to 3 days and then were washed with 10 mM phosphate buffered saline, pH 7.4 (PBS), in order to remove the non-adhered cells. The attached cells were then fixed by incubating the surfaces with a 4% (w/v) paraformaldehyde solution (PFA) in PUBS for 20 min. After fixation, cells were rinsed 3 times with PBS and their membrane was permeabilized by treatment with a 0.1% (v/v) Triton X-100 solution in PBS, for 5 min. After gentle washing with PBS, the cells were incubated with 5 mg/ml BSA solution in PBS (blocking solution) for 1 h at room temperature. Then, the fixed cells were incubated with a 150 nM Phalloidin Atto 488 solution in PBS for 2 h, to visualize cytoskeleton (F-actin), followed by washing 3 times with PBS. Following that, cell nuclei were stained by incubating the surfaces with a 100 ng/mL 4',6'-diamidino-2-phenylindol solution (DAPI) in PBS for 5 min. After washing, the surfaces were observed with an epifluorescence microscope (Axioscop 2, Carl Zeiss) facilitated with appropriate excitation/emission filter pairs (for DAPI 365/420 nm, for Phalloidin Atto488 485/515 mn). Cell adhesion experiments were performed at least three times in duplicate.

In Figure 1, SEM images of PMMA surfaces treated with bias (a) -50 V, (b) - 75 V, (c) -100 V for 1 min, and (d) -100 Volt for 3 main are presented. The mean height of the nanostructures are (a) 85 nm, (b) 140 nm (c) 380 nm, and (d) 800 nm and the rms are (a) 8,7 nm, (b) 29,6 nm, (c) 46,8 nm, (d) 150 nm. It is observed that the nanostructures of the surfaces become higher as the bias increases. Also for a set bias voltage value (-100 V), when the etching time increases (from 1 to 3 min), the structures merge together to larger hierarchical structures. Fig. 1e shows a top down view of fig. 1d, showing the hierarchy of the nanostructures at -100V, 3min etching and the merging together of various nanostructures.

Figure 2 presents in columnar (bar format) the cancer and normal cell populations per surface area for untreated and O₂ plasma treated surfaces for a) 1 day and b) 3 days culture, respectively (conditions O₂ plasma, 0V, -25V, -50V -75V, -100V for 1 min and -100V for 3 min). As can be seen from Fig 2, the cancer cells adhere poorly on untreated PMMA surfaces, compared to all plasma treated ones after one day culture. On the other hand, the normal fibroblasts adhere more on the untreated surfaces compared to the treated ones. After 3 days of culture, it appears that the number of adhered cancer cells is still very low on untreated surfaces, compared to the treated ones. At the same time, a greater number of normal fibroblasts adheres on the untreated surface compared to 1-day culture. Furthermore, the population of normal cells after 3-day culture was considerably decreased compared to 1-day culture. Especially concerning the cancer cells, their population on nanotextured surfaces, prepared using -100V bias voltage for 3 min, was increased 6-times after 3 days of culture as compared to 1 day, whereas the normal cells population was reduced by approximately 50% after 3 days of culture as compared to 1 day.

In Figure 3, the proliferation rate of normal and cancer cells for untreated and O₂ plasma treated nanotextured surfaces is shown. As it is demonstrated, normal fibroblasts proliferate almost equally compared to cancer cells on surfaces with less topography (i.e. treated for up to -50 V), while their proliferation rate decreases when the bias voltage employed for surface nanotexturing was equal to or higher than -75V, and reached values less than 1 when the surface was prepared through treatment at -100V bias voltage for 3 min. The opposite behavior was exhibited by cancer fibroblasts, which multiply 12-times faster on the very rough surfaces (-100 V, 3 min).

Concerning cells morphology, normal fibroblasts show the same cytoplasm morphology when grown on either a coverslip or on untreated PMMA surfaces, indicating that the PMMA surface does not affect the cells morphology (see Figure 4a left), whereas, after three days of culture, complete distortion of the morphology of the cytoplasm was observed on the rough PMMA surfaces (see Figure 4a right). On the same surface, the nucleus surface area was shrunk by 30%, in comparison to the nucleus of cells grown on untreated PMMA surface (table 1). Regarding the growth of cancer cells on the studied surfaces, it was found that the cancer cells morphology was not affected at all on the rough surfaces fabricated at -100 V for 3 min after 3 days of culture. Although the number of adhered cells on the untreated PMMA surfaces was very low compared to the treated ones, no noticeable shrinkage in either the cytoplasm (figure 4) or the nucleus (Table 1) was observed, indicating that the untreated surface did not favor their adhesion. Indeed, the cells that eventually adhered, grew on the surface without their morphology being affected.

**Table 1. Nucleus surface area of normal fibroblasts and cancer cells on untreated and plasma treated surfaces after 3 days of culture.**

| | **Mean nucleus surface area ± SD** (**µm²)** | |
|---|---|---|
| | **Normal fibroblasts** | **HT 1080 cell line** |
| **Untreated** | **182(±32)** | **156(±30)** |
| **-100 V, 3min** | **123(±25)** | **155(±15)** |

### Example 2: Enrichment of fibrosarcoma HT1080 cancer cells from a mixture with normal cells on plasma nanotextured substrates

The results obtained through single cancer or normal cells culture on the prepared surfaces led to the conclusion that the adhesion of cancer cells on rough PMMA surfaces was much higher than that of normal cells. For this reason, co-culture of the two different types of cells was performed on untreated and O₂ plasma nanotextured surfaces prepared under optimal conditions for one and three days. At first both normal and cancer cells were treated as described in Example 1. Initial cell solutions containing cancer and normal cells at ratios of 1:10, 1:50 and 1:100 weres prepared and used to seed the surfaces. The total cell number was 2.5x10⁴ cells/mL. As shown in Figure 5a,b,c, after one day co-culture, a greater number of normal fibroblasts adhered on the untreated PMMA surface in comparison to cancer cells (ratio of cancer to normal cell population of: 0.25 when the initial ratio was 0.1, 0.13 when the initial ratio was 0.02 and 0.048 when the initial ratio was 0.01). On the other hand, the O₂ plasma treated surface favored the adhesion of cancer cells against the normal ones, resulting in a ratio of cancer to normal cell population of: 1.8 when the initial ratio was 0.1, 1.5 when the initial ratio was 0.02 and 1.4 when the initial ratio was 0.01, although the number of cancer cells that were initially seeded was from 10 to 100 times less than the normal ones. Thus, a 18-144x enrichment was achieved after 1-day culture.

The enrichment achieved on the nanotextured surfaces was even higher after a 3-day culture (Fig. 5c,d,e), with a ratio of cancer to normal cell population of 3.9 when the initial ratio was 0.1, 2.1 when the initial ratio was 0.02 and 1.4 when the initial ratio was 0.01 (39-144x enrichment). On the contrary, on the untreated surfaces the ratio of cancer to normal cell population was 0.66 when the initial ratio was 0.1, 0.28 when the initial ratio was 0.02 and 0.19 when the initial ratio was 0.01, due to decrease of cancer cell population with respect to normal ones (Table 2).

From the above results, it can be noted that the adhesion of cancer and normal cells depends on the nanotexture of the surfaces; and significant amplification (enrichment by a factor larger than 140:1) can be achieved on the nanotextured surfaces with the high aspect ratio topography, when the cancer cells are 100 times less than the normal cells in the initial solution.

**Table 2. Ratios of cancer cells to normal cells and enrichment ratios for 1 and 3 days of co-culture**

| | Initial ratio | 1 day | | 3 days | |
|---|---|---|---|---|---|
| | | Ratio | Enrichment ratio | Ratio | Enrichment ratio |
| Flat PMMA | 1/10 | 0.25 | 2.5 | 0.66 | 6.6 |
| | 1/50 | 0.13 | 6.5 | 0.28 | 14 |
| | 1/100 | 0.048 | 4.8 | 0.18 | 18 |
| 100 V 3min | 1/10 | 1.8 | 18 | 3.9 | 39 |
| | 1/50 | 1.5 | 75 | 2.14 | 107 |
| | 1/100 | 1.4 | 140 | 1.44 | 144 |

### Example 3: Enhanced adhesion and proliferation of PC3 cancer versus mononuclear blood cells on oxygen plasma nanotextured PMMA surfaces

In this example we used PMMA films prepared as described in Example 1. The surface was nanotextured in O₂ plasma (1900 W source power, 0.75 Pa pressure, 100 sccm oxygen flow, 0 to -100 V bias voltage,-and sample temperature during etching 65 °C). To evaluate the adhesion and proliferation of prostate cancer cells of PC3 cancer line with respect to mononuclear cells isolated from peripheral blood, co-culture on flat and nanotextured surfaces was performed. The cells were firstly cultured on standard Petri dishes, detached, and seeded on the surfaces as described in Example 1. The initial number of mononuclear cells in the solution used to seed the PMMA surfaces was 2*10⁵ cells/ml, while the cancer cells were 4 times less (5*10⁴ cells/ml). The cells were co-cultured for 1 day and then the attached to surfaces cells were fixed as described in Example 1. After fixation, the cells were incubated with a solution containing 1 µg/ml, of rabbit polyclonal anti-CD45 antibody (Santa Cruz Biotechnology, Inc.) and 4 µg/ml of mouse monoclonal anti-CK 8,18 antibody (Acris antibodies, Inc.) in PBS containing 5% BSA for 1.5 h, for the staining of mononuclear and PC3 cells respectively. Then the surfaces were washed 3 times with PBS and incubated with a solution containing 5 µg/ml of AlexaFluor 488- labeled anti-rabbit IgG antibody (Invitrogen) and 5 µg/ml of AlexaFluor 546- labeled anti-mouse IgG antibody (Invitrogen) in PBS for 1.5 h, followed by washing 3 times with PBS. Following that, cell nuclei were stained by incubating the surfaces with a 100 ng/mL 4',6'-diamidino-2-phenylindol solution (DAPI) in PBS for 5 min. After washing, the surfaces were observed with an epifluorescence microscope (Axioscop 2, Carl Zeiss) facilitated with appropriate excitation/emission filter pairs (for DAPI 365/420 nm, for AlexaFluor 488 493/520 nm and for AlexaFluor 546 562/573 nm). Cell adhesion experiments were performed three times in duplicate. The cancer cells were discriminated by the normal ones based on their size.

Figure 6 shows SEM-images of PMMA surfaces treated with bias of (a) -50 V, (b) - 75 V, (c) -100 V for 1 min, and (d) -100 V for 3 min. The mean height of the nanostructures are (a) 90 nm, (b) 110 nm, (c) 160 nm, and (d) 400 nm and the rms are (a) 9,6 nm, (b) 17,5 nm, (c) 25,7 nm, (d) 80 nm.

Figure 7 shows in columnar (bar format) the PC3 cell line and the mononuclear cell populations per surface area for untreated and O₂ plasma treated surfaces for 1 day culture. The number of adhered cancer cells increases on the rougher surfaces, while the number of adhered monocytes decreases. Thus, a large difference in the number of adherent cells is observed on the surfaces treated with -100V for 3 min, showing that selective enrichment is achieved for bias voltage equal or higher than -75V, whereas the highest difference was achieved at -100 V bias.

## Claims

1. Use of an organic polymeric surface exhibiting hierarchical nanotexture morphology having an average nanotexture height larger than 140 nm, spaced at least 100 nm apart and exhibiting a water contact angle 0-50°, for the enrichment of cancer cells from a mixture containing cancer and normal cells.

2. Use of an organic polymeric surface according to claim 1, wherein the hierarchical, nanotexture morphology of the organic polymeric surface is produced by an oxygen containing plasma, under anisotropic etching conditions with operating pressure 0.4-7 Pa in the presence of an etching inhibitor, with absolute value of the bias voltage larger than 75V, and plasma processing time larger than 1min.

3. Use of an organic polymeric surface according to claim 1 or 2, without the use of antibodies that recognize specific antigens on the cancer cell membrane.

4. Use of an organic polymeric surface according to claim 1, 2 or 3, wherein the organic polymeric surface is made of a polymer selected from the group consisting of acrylic copolymers, olefin polymers, phenolic polymers, silicones or silicone-organic copolymers.

5. Use of an organic polymeric surface according to claim 4, wherein the polymer is selected from the group consisting of poly ether ether ketone, poly ethylene terepthalate, cyclo olephin polymers, cyclo olephin copolymers, poly styrene, poly dimethyl siloxane, and poly dimethyl siloxane -acrylate copolymers, and preferably poly(methyl methacrylate).

6. Use of an organic polymeric surface according to any one of the preceding claims, wherein the mixture containing cancer and normal cells is a biological sample.

7. Use of an organic polymeric surface according to any one of the preceding claims, wherein the mixture is a tissue sample containing epithelial cancer cells.

8. Use of an organic polymeric surface according to any one of claims 1-6, wherein the mixture is a blood sample containing prostate cancer cells.

9. A diagnostic device for the enrichment of cancer cells from a mixture containing cancer and normal cells comprising an organic polymeric surface exhibiting hierarchical nanotexture morphology having anaverage nanotexture height larger than 140 nm, spaced at least 100 nm apart and exhibiting a water contact angle 0-50 degrees.

10. A diagnostic device according to claim 9, where the hierarchically nanotextured organic polymeric surface is produced by an oxygen containing plasma, under anisotropic etching conditions with operating pressure 0.4-7 Pa in the presence of an etching inhibitor, with absolute value of the bias voltage larger than 75V, and plasma processing time larger than 1min.

## Patentansprüche

1. Verwendung einer organischen polymeren Oberfläche, welche eine Morphologie hierarchischer Nanotextur vorweist, mit einer durchschnittlichen Höhe der Nanostrukturen größer als 140 nm, einem Abstand zueinander von mindestens 100 nm und einem Kontaktwinkel von 0-50°, zur Anreicherung von Tumorzellen aus einer Mischung, welche Krebs- und normale Zellen beinhaltet.

2. Verwendung einer organischen polymeren Oberfläche nach Anspruch 1, wobei die Morphologie hierarchischer Nanotextur durch Ätzen in einem Sauerstoff enthaltenden Plasma entsteht, unter anisotropen Ätzbedingungen mit 0,4-7 Pa Arbeitsdruck, in Gegenwart von Ätzinhibitoren, einem absolutem Potentialwert der Vorspannung größer als 75V und einer Behandlungszeit mit dem Plasma von mehr als 1 min.

3. Verwendung einer organischen polymeren Oberfläche nach den Ansprüchen 1 oder 2, ohne die Verwendung von Antikörpern, welche spezielle Membranantigene von Krebszellen erkennen.

4. Verwendung einer organischen polymeren Oberfläche nach einem der Ansprüche 1, 2 oder 3, wobei die organische polymere Oberfläche aus einem Polymer hergestellt wird, ausgewählt aus einer Gruppe, welche aus Acrylcopalymeren, olefinischen Polymeren, phenolischen Polymeren, Silikonen oder organischen Silicon-Copolymeren besteht.

5. Verwendung einer organischen polymeren Oberfläche nach Anspruch 4, wobei das Polymer aus der Gruppe ausgewählt wird, welche aus Poly ether ether ketone, Poly ethylenterephthalat, kyklolefinis Polymer, Copolymer kyklolefinis, Polystyrol, Poly dimethylsiloxan und Acrylcopolymere Poly dimethylsiloxan, vorzugsweise Poly methylmethacrylat besteht.

6. Verwendung einer organischen polymeren Oberfläche nach einem der vorhergehenden Ansprüche, wobei die Mischung, welche Krebs- und normale Zellen beinhaltet, eine biologische Probe ist.

7. Verwendung einer organischen polymeren Oberfläche nach einem der vorhergehenden Ansprüche, wobei die Mischung eine Probe von Gewebe ist, welche epitheliale Krebszellen enthält.

8. Verwendung einer organischen polymeren Oberfläche nach den Ansprüchen 1-6, wobei die Mischung eine Blutprobe ist, welche Prostatakrebszellen enthält.

9. Eine Diagnosevorrichtung zur Anreicherung von Tumorzellen aus einer Mischung, welche Krebs- und normale Zellen enthält, welche aus einer organischen polymeren Oberfläche, mit einer Morphologie hierarchischer Nanotextur, mit einer durchschnittlichen Höhe der Nanostrukturen größer als 140 nm, einem Abstand zueinander von mindestens 100 nm und einem Kontaktwinkel von 0-50°, besteht.

10. Eine Diagnosevorrichtung nach dem Ansprüch 9, bei welcher die Morphologie hierarchischer Nanotextur durch Ätzen in einem Sauerstoff enthaltenden Plasma entsteht, unter anisotropen Ätzbedingungen mit 0,4-7 Pa Arbeitsdruck, in Gegenwart von Ätzinhibitoren, einem absolutem Potentialwert der Vorspannung größer als 75V und einer Behandlungszeit mit dem Plasma von mehr als 1 min.

## Revendications

1. . L'utilisation d'une surface de polymère organique présentant une morphologie à nanotexture hiérarchique, ayant des nanotextures de hauteur moyenne supérieure à 140 nm, espacées d'un écart type d'au moins 100 nm et présentant un angle de contact avec l'eau compris entre 0 et 50°, pour l'enrichissement de cellules cancéreuses à partir d'un mélange contenant des cellules cancéreuses et des cellules saines.

2. . L'utilisation d'une surface de polymère organique selon la revendication 1, dans laquelle la morphologie à nanotexture hiérarchique de la surface du polymère organique est produite par un plasma contenant de l'oxygène, sous des conditions anisotropes de gravure à une pression de fonctionnement de 0.4 à 7 Pa en présence d'un inhibiteur de gravure, à une valeur absolue de tension de polarisation supérieure à 75 V, et un temps de traitement au plasma supérieur à 1min.

3. . L'utilisation d'une surface de polymère organique selon la revendication 1 ou 2, sans l'utilisation d'anticorps qui reconnaissent des antigènes spécifiques sur la membrane des cellules cancéreuses.

4. . L'utilisation d'une surface de polymère organique selon la revendication 1,2 ou 3, dans laquelle la surface de polymère organique est faite d'un polymère choisi dans le groupe constitué par les copolymères acryliques, les polymères d'oléfines, les polymères phénoliques, les silicones ou les copolymères de silicone-organique.

5. . L'utilisation d'une surface de polymère organique selon la revendication 4, dans laquelle le polymère est choisi dans le groupe constitué par le poly éther éther cétone, le poly éthylène téréphtalate, les polymères de cyclo oléfines, les copolymères de cyclo oléfines, le poly styrène, le poly diméthylsiloxane, et les copolymères de poly diméthylsiloxane et d'acrylate, et de préférence le poly (méthacrylate de méthyle).

6. . L'utilisation d'une surface de polymère organique selon l'une quelconque des revendications précédentes, dans laquelle le mélange contenant des cellules cancéreuses et saines est un échantillon biologique.

7. . L'utilisation d'une surface de polymère organique selon l'une quelconque des revendications précédentes, dans laquelle le mélange est un échantillon de tissu contenant des cellules cancéreuses épithéliales.

8. . L'utilisation d'une surface de polymère organique selon l'une quelconque des revendications 1-6. dans laquelle le mélange est un échantillon de sang contenant des cellules cancéreuses de la prostate.

9. . Un dispositif de diagnostic pour l'enrichissement de cellules cancéreuses à partir d'un mélange contenant des cellules cancéreuses et des cellules saines comprenant une surface de polymère organique présentant une morphologie à nanotexture hiérarchique ayant des nanotexture de hauteur moyenne supérieure à 140 nm, espacées d'un écart type d'au moins 100 nm et présentant un angle de contact avec l'eau compris entre 0 et 50 degrés.

10. . Le dispositif de diagnostic selon la revendication 9, dans lequel la surface de polymère organique hiérarchiquement nanotexturée est produite par un plasma contenant de l'oxygène, sous des conditions anisotropes de gravure à une pression de fonctionnement de 0.4 à 7 Pa en présence d'un inhibiteur de gravure, à une valeur absolue de tension de polarisation supérieure à 75 V, et un temps de traitement au plasma supérieur à 1 min.
